# EUROPEAN PATENT APPLICATION

(11) **EP 1 482 050 A1**
(43) Date of publication of application: **01.12.2004**
(21) Application number: 03447133.4
(22) Date of filing: 28.05.2003
(51) Int. Cl.: C12N 15/56, C12N 15/70, C12N 9/42, C12N 1/19, C12N 1/21, A21D 8/04, A23L 2/84, C02F 3/34

(54) **Enzyme with xylanase activity at acidic ph**

(71) Applicant: Facultés Universitaires Notre-Dame de la Paix, 5000 Namur (BE)
(72) Inventor: Housen, Isabelle, 5024 Gelbressée (BE); Coppe, Philippe, 6900 Aye (BE); Vandenhaute, Jean, 5100 Wierde (BE); Depiéreux, Eric, 5000 Namur (BE)
(74) Representative: Van Malderen, Joelle

(57) **Abstract**

The present invention is related to an isolated and purified enzyme with xylanolytic activity having more than 70% of sequence identity with the amino acid sequence SEQ ID NO 8.

SEQUENCE LISTING

<110>  FACULTES UNIVERSITAIRES NOTRE-DAME DE LA PAIX

<120>  Enzyme with Xylanase Activity at Acidic pH

<130>  P.FNDP.15/EP

<140>  EP 03447133.4
<141>  2003-05-28

<160>  100

<170>  PatentIn version 3.2

<210>  1
<211>  201
<212>  PRT
<213>  Aureobasidium pullulans

<400>  1

Ala Gly Pro Gly Gly Ile Asn Tyr Val Gln Asn Tyr Asn Gly Asn Leu
1               5                   10                  15

Gly Gln Phe Thr Tyr Asn Glu Asn Ala Gly Thr Tyr Ser Met Tyr Trp
            20                  25                  30

Asn Asn Gly Val Asn Gly Asp Phe Val Val Gly Leu Gly Trp Ser Thr
        35                  40                  45

Gly Ala Ala Arg Ser Ile Thr Tyr Ser Ser Asn Tyr Gln Ala Ser Gly
    50                  55                  60

Gly Ser Tyr Leu Ser Val Tyr Gly Trp Ile Asn Ser Pro Gln Ala Glu
65                  70                  75                  80

Tyr Tyr Ile Val Glu Ser Tyr Gly Ser Tyr Asn Pro Cys Gly Ala Gly
                85                  90                  95

Gln Ser Gly Val Thr Gln Ser Tyr Asn Pro Cys Gly Ala Gly Gln Ser
            100                 105                 110

Gly Val Thr Gln Leu Gly Thr Val Cys Ser Asp Gly Ala Thr Tyr Thr
        115                 120                 125

Val Tyr Thr Asp Thr Arg Thr Asn Gln Pro Ser Ile Thr Gly Thr Ser
    130                 135                 140

Thr Phe Lys Gln Tyr Trp Ser Val Arg Gln Thr Lys Arg Thr Ser Gly
145                 150                 155                 160

Thr Val Thr Thr Gly Asn His Phe Ala Tyr Trp Ala Lys Tyr Gly Phe
                165                 170                 175

Gly Asn Ser Tyr Asn Phe Gln Val Met Pro Val Glu Ala Phe Ser Gly
            180                 185                 190

Thr Gly Ser Ala Ser Val Thr Val Ser
        195                 200

<210>  2
<211>  195
<212>  PRT
<213>  Penicillium purporogenum

<400>  2

Ser Ile Asn Tyr Val Gln Asn Tyr Asn Gly Asn Leu Gly Ala Phe Ser
1               5                   10                  15

Tyr Asn Glu Gly Ala Gly Thr Phe Ser Met Tyr Trp Gln Gln Gly Val
            20                  25                  30

Ser Asn Asp Phe Val Val Gly Leu Gly Arg Ser Thr Gly Ser Ser Asn
        35                  40                  45

Pro Ile Thr Tyr Ser Ala Ser Tyr Ser Ala Ser Gly Gly Ser Tyr Leu
    50                  55                  60

Ala Val Tyr Gly Trp Val Asn Ser Pro Gln Ala Glu Tyr Tyr Val Val
65                  70                  75                  80

Glu Ala Tyr Gly Asn Tyr Asn Pro Cys Ser Ser Gly Ser Ala Thr Asn
                85                  90                  95

Asn Tyr Asn Pro Cys Ser Ser Gly Ser Ala Thr Asn Leu Gly Thr Val
            100                 105                 110

Ser Ser Asp Gly Gly Thr Tyr Gln Val Cys Thr Asp Thr Arg Val Asn
        115                 120                 125

Gln Pro Ser Ile Thr Gly Thr Ser Thr Phe Thr Gln Phe Phe Ser Val
    130                 135                 140

Arg Gln Gly Ser Arg Thr Ser Gly Thr Val Thr Ile Ala Asn His Phe
145                 150                 155                 160

Asn Phe Trp Ala Asn Asp Gly Phe Gly Asn Ser Asn Phe Asn Tyr Gln
                165                 170                 175

Val Val Ala Val Glu Ala Trp Ser Gly Thr Gly Thr Ala Ser Val Thr
            180                 185                 190

Val Ser Ala
        195

<210>  3
<211>  206
<212>  PRT

<213>  Aspergillus niger

<400>  3

Ala Pro Ala Pro Glu Pro Val Leu Val Ser Arg Ser Ala Gly Ile Asn
1               5                   10                  15

Tyr Val Gln Asn Tyr Asn Gly Asn Leu Gly Asp Phe Thr Tyr Asp Glu
            20                  25                  30

Ser Thr Gly Thr Phe Ser Met Tyr Trp Glu Asp Gly Val Ser Ser Asp
        35                  40                  45

Phe Val Val Gly Leu Gly Trp Thr Thr Gly Ser Ser Lys Ser Ile Thr
    50                  55                  60

Tyr Ser Ala Gln Tyr Ser Ala Ser Ser Ser Ser Ser Tyr Leu Ala Val
65                  70                  75                  80

Tyr Gly Trp Val Asn Ser Pro Gln Ala Glu Tyr Tyr Ile Val Glu Asp
                85                  90                  95

Tyr Gly Asp Tyr Asn Pro Cys Ser Ser Ala Thr Ser Leu Asp Tyr Asn
            100                 105                 110

Pro Cys Ser Ser Ala Thr Ser Leu Gly Thr Val Tyr Ser Asp Gly Ser
        115                 120                 125

Thr Tyr Gln Val Cys Thr Asp Thr Arg Arg Thr Arg Pro Ser Ile Thr
    130                 135                 140

Gly Thr Ser Thr Phe Thr Gln Tyr Phe Ser Val Arg Glu Ser Thr Arg
145                 150                 155                 160

Thr Ser Gly Thr Val Thr Ile Ala Asn His Phe Asn Phe Trp Ala Gln
                165                 170                 175

His Gly Phe Gly Asn Ser Asn Phe Asn Tyr Gln Val Met Ala Val Glu
            180                 185                 190

Ala Trp Asn Gly Val Gly Ser Ala Ser Val Thr Ile Ser Ser
        195                 200                 205

<210>  4
<211>  195
<212>  PRT
<213>  Aspergillus tubingensis

<400>  4

Ser Ala Gly Ile Asn Tyr Val Gln Asn Tyr Asn Gly Asn Leu Gly Asp
1               5                   10                  15

Phe Thr Tyr Asp Glu Ser Ala Gly Thr Phe Ser Met Tyr Trp Glu Asp

20                  25                  30

Gly Val Ser Ser Asp Phe Val Val Gly Leu Gly Trp Thr Thr Gly Ser
        35                  40                  45

Ser Asn Ala Ile Thr Tyr Ser Ala Glu Tyr Ser Ala Ser Gly Ser Ala
    50                  55                  60

Ser Tyr Leu Ala Val Tyr Gly Trp Val Asn Tyr Pro Gln Ala Glu Tyr
65                  70                  75                  80

Tyr Ile Val Glu Asp Tyr Gly Asp Tyr Asn Pro Cys Ser Ser Ala Thr
                85                  90                  95

Ser Leu Asp Tyr Asn Pro Cys Ser Ser Ala Thr Ser Leu Gly Thr Val
            100                 105                 110

Tyr Ser Asp Gly Ser Thr Tyr Gln Val Cys Thr Asp Thr Arg Thr Asn
        115                 120                 125

Glu Pro Ser Ile Thr Gly Thr Ser Thr Phe Thr Gln Tyr Phe Ser Val
    130                 135                 140

Arg Glu Ser Thr Arg Thr Ser Gly Thr Val Thr Val Ala Asn His Phe
145                 150                 155                 160

Asn Phe Trp Ala His His Gly Phe Gly Asn Ser Asp Phe Asn Tyr Gln
                165                 170                 175

Val Val Ala Val Glu Ala Trp Ser Gly Ala Gly Ser Ala Ser Val Thr
            180                 185                 190

Ile Ser Ser
        195

<210>  5
<211>  195
<212>  PRT
<213>  Aspergillus niger

<400>  5

Ser Ala Gly Ile Asn Tyr Val Gln Asn Tyr Asn Gly Asn Leu Gly Asp
1               5                   10                  15

Phe Thr Tyr Asp Glu Ser Ala Gly Thr Phe Ser Met Tyr Trp Glu Asp
            20                  25                  30

Gly Val Ser Ser Asp Phe Val Val Gly Leu Gly Trp Thr Thr Gly Ser
        35                  40                  45

Ser Lys Ala Ile Thr Tyr Ser Ala Glu Tyr Ser Ala Ser Gly Ser Ser
    50                  55                  60

Ser Tyr Leu Ala Val Tyr Gly Trp Val Asn Tyr Pro Gln Ala Glu Tyr
65                  70                  75                  80

Tyr Ile Val Glu Asp Tyr Gly Asp Tyr Asn Pro Cys Ser Ser Ala Thr
                85                  90                  95

Ser Leu Asp Tyr Asn Pro Cys Ser Ser Ala Thr Ser Leu Gly Thr Val
            100                 105                 110

Tyr Ser Asp Gly Ser Thr Tyr Gln Val Cys Thr Asp Thr Arg Thr Asn
        115                 120                 125

Glu Pro Ser Ile Thr Gly Thr Ser Thr Phe Thr Gln Tyr Phe Ser Val
    130                 135                 140

Arg Glu Ser Thr Arg Thr Ser Gly Thr Val Thr Val Ala Asn His Phe
145                 150                 155                 160

Asn Phe Trp Ala Gln His Gly Phe Gly Asn Ser Asp Phe Asn Tyr Gln
                165                 170                 175

Val Met Ala Val Glu Ala Trp Ser Gly Ala Gly Ser Ala Ser Val Thr
            180                 185                 190

Ile Ser Ser
        195

<210>  6
<211>  195
<212>  PRT
<213>  Aspergillus niger var. awamorii

<400>  6

Ser Ala Gly Ile Asn Tyr Val Gln Asn Tyr Asn Gly Asn Leu Gly Asp
1               5                   10                  15

Phe Thr Tyr Asp Glu Ser Ala Gly Thr Phe Ser Met Tyr Trp Glu Asp
            20                  25                  30

Gly Val Ser Ser Asp Phe Val Val Gly Leu Gly Trp Thr Thr Gly Ser
        35                  40                  45

Ser Asn Ala Ile Thr Tyr Ser Ala Glu Tyr Ser Ala Ser Gly Ser Ser
    50                  55                  60

Ser Tyr Leu Ala Val Tyr Gly Trp Val Asn Tyr Pro Gln Ala Glu Tyr
65                  70                  75                  80

Tyr Ile Val Glu Asp Tyr Gly Asp Tyr Asn Pro Cys Ser Ser Ala Thr
                85                  90                  95

Ser Leu Asp Tyr Asn Pro Cys Ser Ser Ala Thr Ser Leu Gly Thr Val
            100                 105                 110

Tyr Ser Asp Gly Ser Thr Tyr Gln Val Cys Thr Asp Thr Arg Thr Asn
        115                 120                 125

Glu Pro Ser Ile Thr Gly Thr Ser Thr Phe Thr Gln Tyr Phe Ser Val
    130                 135                 140

Arg Glu Ser Thr Arg Thr Ser Gly Thr Val Thr Val Ala Asn His Phe
145                 150                 155                 160

Asn Phe Trp Ala Gln His Gly Phe Gly Asn Ser Asp Phe Asn Tyr Gln
                165                 170                 175

Val Met Ala Val Glu Ala Trp Ser Gly Ala Gly Ser Ala Ser Val Thr
            180                 185                 190

Ile Ser Ser
        195

<210>  7
<211>  195
<212>  PRT
<213>  Aspergillus kawachii

<400>  7

Ser Ala Gly Ile Asn Tyr Val Gln Asn Tyr Asn Gly Asn Leu Gly Asp
1               5                   10                  15

Phe Thr Tyr Asp Glu Ser Ala Gly Thr Phe Ser Met Tyr Trp Glu Asp
            20                  25                  30

Gly Val Ser Ser Asp Phe Val Val Gly Leu Gly Trp Thr Thr Gly Ser
        35                  40                  45

Ser Asn Ala Ile Ser Tyr Ser Ala Glu Tyr Ser Ala Ser Gly Ser Ser
    50                  55                  60

Ser Tyr Leu Ala Val Tyr Gly Trp Val Asn Tyr Pro Gln Ala Glu Tyr
65                  70                  75                  80

Tyr Ile Val Glu Asp Tyr Gly Asp Tyr Asn Pro Cys Ser Ser Ala Thr
                85                  90                  95

Ser Leu Asp Tyr Asn Pro Cys Ser Ser Ala Thr Ser Leu Gly Thr Val
            100                 105                 110

Tyr Ser Asp Gly Ser Thr Tyr Gln Val Cys Thr Asp Thr Arg Thr Asn
        115                 120                 125

Glu Pro Ser Ile Thr Gly Thr Ser Thr Phe Thr Gln Tyr Phe Ser Val
    130                 135                 140

Arg Glu Ser Thr Arg Thr Ser Gly Thr Val Thr Val Ala Asn His Phe
145                 150                 155                 160

Asn Phe Trp Ala Gln His Gly Phe Gly Asn Ser Asp Phe Asn Tyr Gln
                165                 170                 175

Val Met Ala Val Glu Ala Trp Ser Gly Ala Gly Ser Ala Ser Val Thr
            180                 185                 190

Ile Ser Ser
        195

<210>  8
<211>  205
<212>  PRT
<213>  Scythalidium acidophilum

<400>  8

Met Lys Phe Thr Leu Thr Ile Ala Gly Leu Leu Ala Val Gly Ser Thr
1               5                   10                  15

Ala Ala Pro Thr Thr Glu Lys Arg Asn Pro Gly Gly Ile Asp Tyr Val
            20                  25                  30

Gln Asn Tyr Asn Gly Asp Val Ala Asp Phe Gln Tyr Asn Glu Gly Ala
        35                  40                  45

Gly Thr Tyr Thr Cys Gly Trp Asp Gly Ser Thr Asp Phe Val Val Gly
    50                  55                  60

Leu Gly Trp Ser Thr Gly Ala Ala Arg Asp Ile Thr Tyr Ser Ala Thr
65                  70                  75                  80

Tyr Asn Ala Gly Gly Ser Gly Ser Tyr Leu Ala Val Tyr Gly Trp Val
                85                  90                  95

Asn Ser Pro Gln Ala Glu Tyr Tyr Ile Val Glu Ser Tyr Gly Asp Tyr
            100                 105                 110

Asn Pro Cys Ser Asn Ala Glu Gly Leu Gly Thr Leu Glu Ser Asp Gly
        115                 120                 125

Ser Thr Tyr Thr Val Cys Thr Asp Thr Arg Thr Asn Glu Pro Ser Ile
    130                 135                 140

Thr Gly Thr Ser Thr Phe Thr Gln Tyr Trp Ser Val Arg Gln Ser Glu
145                 150                 155                 160

Arg Thr Ser Gly Thr Val Thr Val Gly Asn His Phe Asn Tyr Trp Ala

165                 170                 175

Gln His Gly Phe Gly Asp Ser Tyr Asn Phe Gln Val Met Ala Val Glu
            180                 185                 190

Ala Phe Ser Gly Ser Gly Ser Ala Ser Val Ser Val Ser
        195                 200                 205

<210>  9
<211>  196
<212>  PRT
<213>  Cryptococcus sp.

<400>  9

Thr Gly Asn Tyr Val Gln Asn Tyr Asn Gly Asn Val Ala Asn Phe Glu
1               5                   10                  15

Tyr Ser Gln Tyr Asp Gly Thr Phe Ser Val Asn Trp Asn Gly Asn Thr
            20                  25                  30

Asp Phe Val Cys Gly Leu Gly Trp Thr Val Gly Thr Gly Arg Thr Ile
        35                  40                  45

Thr Tyr Ser Gly Ser Tyr Asn Pro Gly Tyr Ser Gly Ser Tyr Gln Ala
    50                  55                  60

Ile Tyr Gly Trp Thr Gly Gln Gly Ser Leu Ser Glu Tyr Tyr Val Ile
65                  70                  75                  80

Asp Asn Tyr Gly Gly Tyr Asn Pro Cys Thr Gly Ser Gly Val Thr Gln
                85                  90                  95

Gly Tyr Asn Pro Cys Thr Gly Ser Gly Val Thr Gln Leu Gly Ser Leu
            100                 105                 110

Tyr Ser Asp Gly Ser Ser Tyr Gln Val Cys Thr His Thr Gln Tyr Asn
        115                 120                 125

Gln Pro Ser Ile Val Gly Thr Thr Thr Phe Pro Gln Tyr Phe Ser Val
    130                 135                 140

Arg Gln Asn Lys Arg Ser Ser Gly Ser Val Asn Met Gln Asn His Phe
145                 150                 155                 160

Asn Tyr Trp Ala Gln His Gly Phe Pro Asn Arg Asn Phe Asn Tyr Gln
                165                 170                 175

Val Leu Ala Val Glu Gly Phe Ser Gly Ser Gly Asn Ala Asn Met Lys
            180                 185                 190

Leu Ile Ser Gly
        195

<210> 10
<211> 17
<212> DNA
<213> Artificial Sequence

<220>
<223> primer


<220>
<221> misc_feature
<222> (3)..(3)
<223> n is a, c, g, or t

<400> 10
gcngartayt ayathgt 17


<210> 11
<211> 1550
<212> DNA
<213> Scythalidium acidophilum

<400> 11
agactgtcca gtaatgagga agtgcctctc gcttgattat gtgtagttag gaaaaagact 60

ctccatggcc atatattgcc ggaggtcacc aggcacacac aatatgtata tgtagcgaag 120

tagctaaagc tcaccaaggc tgcaggatcc gggtgatttt catggaggtg gcgctctgga 180

gagcagcagg gctgataatt tcccgggcga gtcggttgat ggtgatcagc gagttgcatg 240

cagttggaga gggcggttaa aggcaagaaa caatctatga acatcatccg gttggtgctt 300

ctcaacagtc taatgcaatc gtgtggaatc ccataaggtg gtgcttactc tacgcaacta 360

tgtcggctaa atatcctcat tgtttcgggt ttgtagcaac ctcgtttcgg acgcctcgga 420

acttcgacat tagtgggacc tatgcctcgc tatttgatac atatagattg gtttgagtga 480

cacggtcaca attttgactg tagctcactt cactaaagcg caccacatgt tcggataaat 540

cgatctggaa tggtgcagtc tagacaaatg gagaaaggga aaaatagtag acaaagcaca 600

gcagttctga atcacacaca tgggtccagg ataaatgaca gatatttgac tagaagcaaa 660

atgaggatat aagagacgcc ggacttcata ttggagaagt ctgactatca actctcttac 720

atcaaaactc aacattttct taccaactgt ttaacacact gatctcccga ttcccaattc 780

aatccccatc atgaagttta cattgaccat tgccggtctc ttggccgttg gctccactgc 840

tgccccgacc accgagaaac gcaatccagg agggattgac tacgtgcaaa actacaacgg 900

cgatgttgcc gacttccagt acaacgaggg tgccggcacc tacacttgcg gctgggatgg 960

ctctactgac tttgtcgttg gccttggttg gtcgaccggt gctgcccggt aagctgaggc 1020

gcagtcgacc tcaagattgg accgcctgtt gacagattcc cagcgatata acttactctg 1080

ctacatacaa tgctggggga tccggttctt accttgccgt atacggctgg gtgaactcac 1140

cccaggcaga gtactatatt gttgaatcat acggcgacta caatccatgt tccaatgctg 1200

aaggtctcgg aacgctcgaa tctgatggaa gcacttacac ggtctgcacc gacacccgca 1260

ccaatgaacc gtccatcacc gggaccagca ccttcacgca atattggtca gttcgtcaga 1320

gcgaacgcac ctcgggcacc gttactgttg gcaaccactt caattactgg gcgcaacacg 1380

ggttcggtga ctcctacaac ttccaggtta tggctgtcga ggctttctct ggctctggaa 1440

gtgccagtgt ctcggtctct tagtctcaaa aaaattgatc gaccgatggg cagtggtttg 1500

gtcaattgat cgaccgatgg gcagtggttt ggtcaattga cggtttggca 1550

<210> 12
<211> 217
<212> DNA
<213> Artificial Sequence

<220>
<223> 5' exon

<400> 12
atgaagttta cattgaccat tgccggtctc ttggccgttg gctccactgc tgcccgacca 60

ccgagaaacg caatccagga gggattgact acgtgcaaaa ctacaacggc gatgttgccg 120

acttccagta caacgagggt gccggcacct acacttgcgg ctgggatggc tctactgact 180

ttgtcgttgg ccttggttgg tcgaccggtg ctgcccg 217

<210> 13
<211> 400
<212> DNA
<213> Artificial Sequence

<220>
<223> 3' exon

<400> 13
cgatataact tactctgcta catacaatgc tgggggatcc ggttcttacc ttgccgtata 60

cggctgggtg aactcacccc aggcagagta ctatattgtt gaatcatacg gcgactacaa 120

tccatgttcc aatgctgaag gtctcggaac gctcgaatct gatggaagca cttacacggt 180

ctgcaccgac acccgcacca atgaaccgtc catcaccggg accagcacct tcacgcaata 240

ttggtcagtt cgtcagagcg aacgcacctc gggcaccgtt actgttggca accacttcaa 300

ttactgggcg caacacgggt tcggtgactc ctacaacttc caggttatgg ctgtcgaggc 360

tttctctggc tctggaagtg ccagtgtctc ggtctcttag 400

<210> 14
<211> 0
<212> DNA
<213>

<400> 14
000

<210> 15
<211> 38
<212> DNA
<213> Artificial Sequence

<220>
<223> synthetic oligonucleotide

<400>  15
atatatatct cgagaaaaga atgaagttta cattgacc                              38


<210>  16
<211>  20
<212>  DNA
<213>  Artificial Sequence

<220>
<223>  synthetic oligonucleotide

<400>  16
cgggcagcac cggtcgacca                                                  20


<210>  17
<211>  21
<212>  DNA
<213>  Artificial Sequence

<220>
<223>  synthetic oligonucleotide

<400>  17
cgatataact tactctgcta c                                                21


<210>  18
<211>  32
<212>  DNA
<213>  Artificial Sequence

<220>
<223>  synthetic oligonucleotide

<400>  18
atatatattc tagactaaga gaccgagaca ct                                    32


<210>  19
<211>  0
<212>  DNA
<213>

<400>  19
000

<210>  20
<211>  17
<212>  DNA
<213>  Artificial Sequence

<220>
<223>  primer


<220>
<221>  misc_feature
<222>  (3)..(3)
<223>  n is a, c, g, or t

<220>
<221>  misc_feature
<222>  (15)..(15)
<223>  n is a, c, g, or t

<400>  20
gcngartayt aygtngt                                                    17


<210>  21
<211>  0
<212>  DNA
<213>

<400>  21
000

<210>  22
<211>  0
<212>  DNA
<213>

<400>  22
000

<210>  23
<211>  0
<212>  DNA
<213>

<400>  23
000

<210>  24
<211>  0
<212>  DNA
<213>

<400>  24
000

<210>  25
<211>  0
<212>  DNA
<213>

<400>  25
000

<210>  26
<211>  0
<212>  DNA
<213>

<400>  26
000

<210>  27
<211>  0
<212>  DNA
<213>

<400>  27
000

<210>  28
<211>  0
<212>  DNA
<213>

<400>  28
000

<210>  29
<211>  0
<212>  DNA
<213>

<400>  29
000

<210>  30
<211>  17
<212>  DNA
<213>  Artificial Sequence

<220>
<223>  primer


<220>
<221>  misc_feature
<222>  (3)..(3)
<223>  n is a, c, g, or t

<220>
<221>  misc_feature
<222>  (15)..(15)
<223>  n is a, c, g, or t

<400>  30
tcngartayt aygtnat                                                    17


<210>  31
<211>  0
<212>  DNA
<213>

<400>  31
000

<210>  32
<211>  0
<212>  DNA
<213>

<400>  32
000

<210>  33
<211>  0
<212>  DNA
<213>

<400>  33
000

<210>  34
<211>  0
<212>  DNA
<213>

<400>  34
000

<210>  35
<211>  0
<212>  DNA
<213>

<400>  35
000

<210>  36
<211>  0
<212>  DNA
<213>

<400>  36
000

<210>  37
<211>  0
<212>  DNA
<213>

<400>  37
000

<210>  38
<211>  0
<212>  DNA
<213>

<400>  38
000

<210>  39
<211>  0
<212>  DNA
<213>

<400>  39
000

<210>  40
<211>  17
<212>  DNA
<213>  Artificial Sequence

<220>
<223>  primer


<220>
<221>  misc_feature
<222>  (15)..(15)
<223>  n is a, c, g, or t

<400>  40
agygartayt aygtnat                                                    17


<210>  41
<211>  0
<212>  DNA
<213>

<400>  41
000

<210>  42
<211>  0
<212>  DNA
<213>

<400>  42
000

<210> 43
<211> 0
<212> DNA
<213>

<400> 43
000

<210> 44
<211> 0
<212> DNA
<213>

<400> 44
000

<210> 45
<211> 0
<212> DNA
<213>

<400> 45
000

<210> 46
<211> 0
<212> DNA
<213>

<400> 46
000

<210> 47
<211> 0
<212> DNA
<213>

<400> 47
000

<210> 48
<211> 0
<212> DNA
<213>

<400> 48
000

<210> 49
<211> 0
<212> DNA
<213>

<400> 49
000

<210> 50
<211> 18
<212> DNA
<213> Artificial Sequence

<220>
<223> primer


<220>
<221> misc_feature

<222>  (7)..(7)
<223>  n is a, c, g, or t

<400>  50
ccartangcr aartgrtt                                                    18


<210>  51
<211>  0
<212>  DNA
<213>

<400>  51
000

<210>  52
<211>  0
<212>  DNA
<213>

<400>  52
000

<210>  53
<211>  0
<212>  DNA
<213>

<400>  53
000

<210>  54
<211>  0
<212>  DNA
<213>

<400>  54
000

<210>  55
<211>  0
<212>  DNA
<213>

<400>  55
000

<210>  56
<211>  0
<212>  DNA
<213>

<400>  56
000

<210>  57
<211>  0
<212>  DNA
<213>

<400>  57
000

<210>  58
<211>  0
<212>  DNA
<213>

<400>  58
000

<210>  59
<211>  0
<212>  DNA
<213>

<400>  59
000

<210>  60
<211>  18
<212>  DNA
<213>  Artificial Sequence

<220>
<223>  primer

<400>  60
ccartarttr aartgrtt                                                    18

<210>  61
<211>  0
<212>  DNA
<213>

<400>  61
000

<210>  62
<211>  0
<212>  DNA
<213>

<400>  62
000

<210>  63
<211>  0
<212>  DNA
<213>

<400>  63
000

<210>  64
<211>  0
<212>  DNA
<213>

<400>  64
000

<210>  65
<211>  0
<212>  DNA
<213>

<400>  65
000

<210>  66
<211>  0
<212>  DNA
<213>

<400>  66
000

<210>  67
<211>  0
<212>  DNA
<213>

<400>  67
000

<210>  68
<211>  0
<212>  DNA
<213>

<400>  68
000

<210>  69
<211>  0
<212>  DNA
<213>

<400>  69
000

<210>  70
<211>  18
<212>  DNA
<213>  Artificial Sequence

<220>
<223>  primer

<400>  70
ccaraarttr aartgrtt                                                    18

<210>  71
<211>  0
<212>  DNA
<213>

<400>  71
000

<210>  72
<211>  0
<212>  DNA
<213>

<400>  72
000

<210>  73
<211>  0
<212>  DNA
<213>

<400>  73
000

<210>  74
<211>  0
<212>  DNA
<213>

<400>  74
000

<210>  75
<211>  0
<212>  DNA
<213>

<400>  75
000

<210>  76
<211>  0
<212>  DNA
<213>

<400>  76
000

<210>  77
<211>  0
<212>  DNA
<213>

<400>  77
000

<210>  78
<211>  0
<212>  DNA
<213>

<400>  78
000

<210>  79
<211>  0
<212>  DNA
<213>

<400>  79
000

<210>  80
<211>  18
<212>  DNA
<213>  Artificial Sequence

<220>
<223>  primer


<220>
<221>  misc_feature
<222>  (4)..(4)
<223>  n is a, c, g, or t

<400>  80
ccangcrtcr aartgrtt                                                    18


<210>  81
<211>  0
<212>  DNA
<213>

<400>  81
000

<210> 82
<211> 0
<212> DNA
<213>

<400> 82
000

<210> 83
<211> 0
<212> DNA
<213>

<400> 83
000

<210> 84
<211> 0
<212> DNA
<213>

<400> 84
000

<210> 85
<211> 0
<212> DNA
<213>

<400> 85
000

<210> 86
<211> 0
<212> DNA
<213>

<400> 86
000

<210> 87
<211> 0
<212> DNA
<213>

<400> 87
000

<210> 88
<211> 0
<212> DNA
<213>

<400> 88
000

<210> 89
<211> 0
<212> DNA
<213>

<400> 89
000

<210> 90
<211> 19
<212> DNA

<213>  Artificial Sequence

<220>
<223>  primer

<400>  90
gcggaatact acatcgttg                                                    19


<210>  91
<211>  0
<212>  DNA
<213>

<400>  91
000

<210>  92
<211>  0
<212>  DNA
<213>

<400>  92
000

<210>  93
<211>  0
<212>  DNA
<213>

<400>  93
000

<210>  94
<211>  0
<212>  DNA
<213>

<400>  94
000

<210>  95
<211>  0
<212>  DNA
<213>

<400>  95
000

<210>  96
<211>  0
<212>  DNA
<213>

<400>  96
000

<210>  97
<211>  0
<212>  DNA
<213>

<400>  97
000

<210>  98
<211>  0
<212>  DNA

<213>

<400>  98
000

<210>  99
<211>  0
<212>  DNA
<213>

<400>  99
000

<210>  100
<211>  20
<212>  DNA
<213>  Artificial Sequence

<220>
<223>  primer

<400>  100
ccagtaggcg aaatggttgc                                                    20

## Description

### Field of the invention

The present invention relates to an enzyme with xylanase activity identified by its amino acid and nucleotide sequences and variants thereof.

The present invention relates also to their uses in the pulp and paper industries and in the agrofood.

### Background of the invention

Xylans are heteropolysaccharides which form the major part of the hemicellulose present in the plant biomass.

The backbone of these polysaccharides is a chain of β 1-4 linked xylopyranosyl residues. Many different side groups could bind to these residues like acetyl, arabinosyl and glucuronosyl residues. Phenolic compounds such as ferulic or hydroxycinnamic acids are also involved through ester binding in the cross linking of the xylan chains or in the linkage between xylan and lignin chains for example.

Endoxylanases hydrolyse specifically the backbone of the hemicellulose. In some cases, the side groups may mask the main chain by steric hindrance. Different xylanase activities already described are characterised by their specificity towards their substrate and the length of the oligomers produced.

These differences between the xylanases concerning their properties seem to be partly related to their respective amino acid sequences. Endoxylanases have been classified into two families (F or 10 and G or 11) according to their sequence similarities (Henrissat & Bairoch, Biochem. J., Vol. 293, p. 781 (1993)). The F family of xylanases are larger, more complex as compared to the G family of xylanases. Moreover the F family xylanases produce small oligosaccharides, while the G family xylanases show a higher affinity for unsubstituted xylan. The F family xylanases have a (α/β)₆ barrel fold structure (Dominguez *et al.* Nature Struct. Biol. Vol. 2, p.29-35 (1995)), whereas the G family xylanases are mostly β-sheet and their overall structure resembles that of a right hand (Torronen et al., EMBO J. vol.13, p.2493-2501 (1994)).

Many different microbial genera have been described to produce one or several xylanases. These microbial genera comprise bacteria as well as eukaryotic organisms like yeast or fungi.

Xylanases are used in various industrial areas such as the pulp, paper, feed and bakery industries. Other applications lie in the juice, wine and beer sectors Xylanases could also be used in the wheat separation process. The observed technological effects are, among others, improved bleachability of the pulp, decreased viscosity of the feed or changes in dough characteristics.

In particular, xylanases have been proposed for the pulp and paper industry in a process called "kraft pulp bleaching" for delignification purposes ("Enzymes for pulp and paper processing", eds Jeffries and Viikari; ACS Symp. Ser. vol.655, American Chemical Society, Washington DC (1996); Bajpai, P. Biotechnol. Prog. Vol. 15, p. 147-157, (1999)) as an alternative to chemical bleaching agents such as chlorine and chlorine derivatives which generate by-products toxic for the environment. Xylanases, and in particular G family xylanases, are added to the pulp before the bleaching after hot alkali treatment, the temperature of the pulp being comprised between 60°C and 70°C, and its pH comprised between 10 and 12, that is to say in sub-optimal conditions for their activity, the optimal activity of xylanases being typically comprised between 45°C and 55°C, and pH 5.0 to 7.5.

In order to solve this problem, efforts have been made for finding or making by protein engineering xylanases with improved thermostability and/or improved activity at elevated pH.

Recently, a new bleaching process has been described ("High-consistency ozone- The solution for reduced chemical costs and better enviroment", M. Bokstrom, A. Tuomi, in *Pulp Bleaching, Principles and Practice,* C.W. Dence and D.W. Reeve, Tappi Press, 1996), wherein the pulp is treated at acidic pH, especially between 2.5 and 3.5, and at a temperature of 50°C to 60°C. The performances for said process are higher to the ones of the previous kraft pulp bleaching so that it could be interesting to use xylanases in such a process.

There is therefore a need for xylanases whose biological properties, and in particular whose optimum of activity would be adapted to said new process.

### Definitions

« Polypeptide » refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslational natural processes or may be made by synthetic methods. Modifications include acetylation, acylation, ADP-ribosylation, amidation, covalent attachment of flavin, covalent attachment of a hem moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-linkings, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino of amino acids to proteins such as arginylation, and ubiquitination. See, for instance, PROTEINS - STRUCTURE AND MOLECULAR PROPERTIES, 2^{nd} Ed., T. E. Creighton, W. H. Freeman and Comany, New York, 1993 and Wolt, F., Posttranslational Protein Modifications: Perspectives and Prospects, pp. 1-12 in POSTTRANSLATIONAL COVALENT MODIFICATION OF PROTEINS, B. C. Johnson, Ed., Academic Press, New York, 1983; Seifter et al., "Analysis for protein modifications and nonprotein cofactors", *Meth. Enzymol.* (1990) 182 : 626-646 and Rattan *et al.,* "Protein Synthesis: Posttranslational Modifications and Aging", *Ann NY Acad Sci* (1992) 663 : 48-62.

"Polynucleotide" generally refers to any polyribonucleotide or polydeoxyribonucleotide, which may be unmodified RNA or DNA or modified RNA or DNA. "Polynucleotides" include, without limitation single- and double-stranded DNA, DNA that is a mixture of single- and double-stranded regions, single- and double- stranded RNA, and RNA that is a mixture of single- and double-stranded regions, hybrid molecules comprising DNA and RNA that may be single-stranded or, more typically, double-stranded or a mixture of single- and double-stranded regions. In addition, "Polynucleotide" refers to triple-stranded regions comprising RNA or DNA or both RNA and DNA. The term "Polynucleotide" also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "Polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another reference polypeptide. Generally, differences are limited so that the sequences of the reference polypeptide and the variant are closely similar overall and, in many regions, identical. A variant and reference polypeptide may differ in amino acid sequence by one or more substitutions (preferably conservative), additions and deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis. Variants should retain one or more of the biological activities of the reference polypeptide. For instance, they should have similar antigenic or immunogenic activities as the reference polypeptide. Antigenicity can be tested using standard immunoblot experiments, preferably using polyclonal sera against the reference polypeptide. The immunogenicity can be tested by measuring antibody responses (using polyclonal sera generated against the variant polypeptide) against purified reference polypeptide in a standard ELISA test. Preferably, a variant would retain all of the above biological activities.

"Identity" is a measure of the identity of nucleotide sequences or amino acid sequences. In general, the sequences are aligned so that the highest order match is obtained. "Identify" per se has an art-recognised meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds, Humana Press, New Jersey, 1994; SEQUENCE ANALYSIS IN MOLECULAR BIOLOGY, von Heijne, G., Academic Press, 1987; and SEQUENCE ANALYSIS PRIMER, Gribskov, M. and Devereux, J., eds, M Stockton Press, New York, 1991). While there exist a number of methods to measure identity between two polynucleotide or polypeptide sequences, the term "identity" is well known to skilled artisans (Carillo, H., and Lipton, D., *SIAM J Applied Math* (1998) 48 : 1073). Methods commonly employed to determine identity or similarity between two sequences include, but are not limited to those disclosed in Guide to Huge Computers, Martin J. Bishop, ed., Academic Press, San Diego, 1994, and Carillo, H., and Lipton, D., *SIAM J Applied Math* (1988) 48 : 1073. Methods to determine identity and similarity are codified in computer programs. Preferred computer program methods to determine identity and similarity between two sequences include, but are not limited to, GCG program package (Devereux, J., *et al., J Molec Biol* (1990) 215 : 403). Most preferably, the program used to determine identity levels was the GAP program, as was used in the Examples hereafter.

As an illustration, by a polynucleotide having a nucleotide sequence having at least, for example, 95% "identity" to a reference nucleotide sequence is intended that the nucleotide sequence of the polynucleotide is identical to the reference sequence except that the polynucleotide sequence may include an average up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5' or 3' terminal positions of the reference nucleotide sequence or anywhere between those terminal positions, interspersed either individually among nucleotides in the reference sequence or in one or more contiguous groups within the reference sequence.

A fragment may be "free-standing" or comprised within a larger polypeptide of which they form a part or region, most preferably as a single continuous region. Representative examples of polypeptide fragments of the invention, include, for example, fragments from about amino acid number 1-30, 31-60, 61-90, 91-120, 121-150, and 150 to the end of the polypeptide. In this context "about" includes the particularly recited ranges larger or smaller by several, 5, 4, 3, 2 or 1 amino acid at either extreme or at both extremes.

Preferred fragments include, for example, truncated polypeptides having the amino acid sequence of the polypeptides, except for deletion of a continuous series of residues that includes the amino terminus, or a continuous series of residues that includes the carboxyl terminus and / or transmembrane region or deletion of two continuous series of residues, one including the amino terminus and one including the carboxyl terminus. Also preferred are fragments characterised by structural or functional attributes such as fragments that comprise alpha-helix and alpha-helix forming regions, beta-sheet and beta-sheet forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments.

### Summary of the invention

A first aspect of the present invention is related to an isolated and purified (from possible contaminants) polypeptide having an amino acid sequence presenting more than 60%, preferably more than 70%, 80% or 85%, more preferably more than 90%, 95% or 98-99% homology (or sequence identity) with the amino acid sequence SEQ ID NO 8.

Advantageously, the isolated and purified polypeptide according to the invention has a molecular weight comprised between 18 and 24 kD, preferably a molecular weight about 19 kD.

Said amino acid sequence or peptide is extracellular or intra-cellular expressed and/or secreted by the recombinant host cell according to the invention.

According to another preferred embodiment of the present invention, the isolated and purified polypeptide is a xylanase which has the amino acid sequence of SEQ ID NO 8, a variant or a portion of said amino acid sequence (of more than 30 or 50 amino-acids, preferably more than 100 amino-acids), which has at least more than 80% of the xylanase activity of the complete amino acid sequence SEQ ID NO 8, preferably more than 95% of the xylanase activity or the complete xylanase activity of the complete amino acid sequence SEQ ID NO 8 (see also example 1 for the measurement of the xylanase activity).

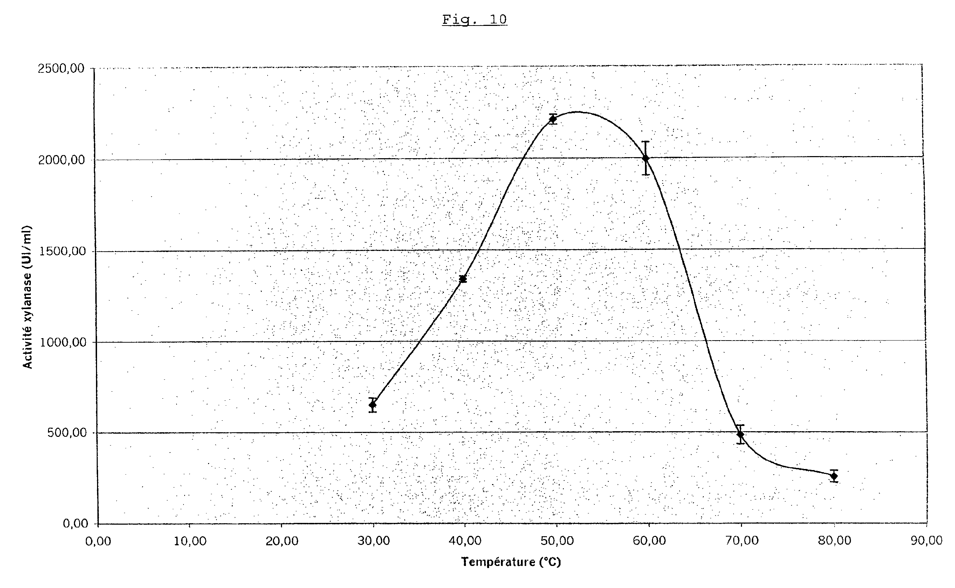

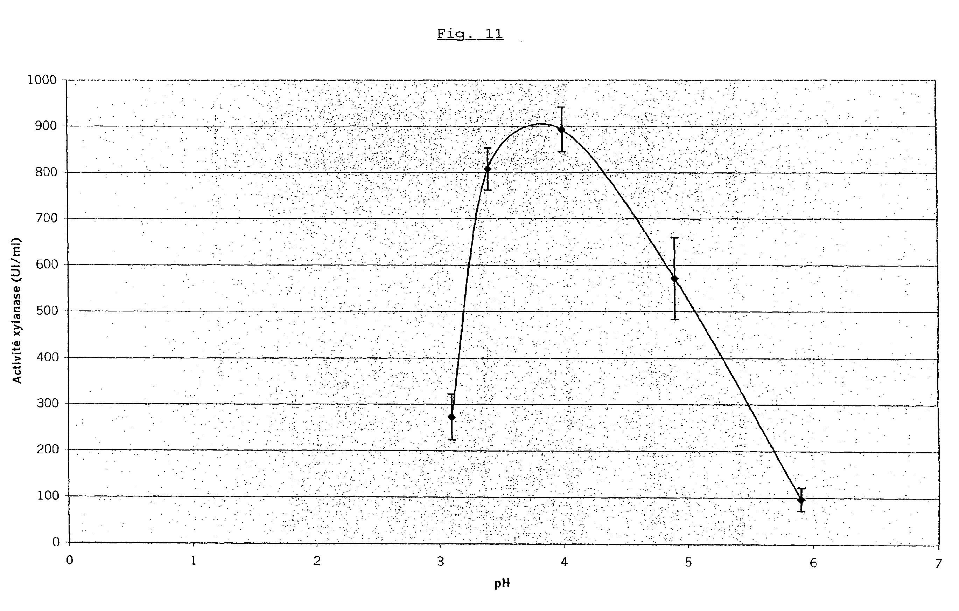

The purified xylanase polypeptide according to the invention is also characterised by an optimum pH for the enzymatic activity around 5.0 and a temperature profile for the enzymatic activity having its maximum at about 50 °C. More generally, the maximum activity of the enzyme is comprised between about pH 3.5 and about 4.5, (see Fig. 11) at a temperature comprised between about 45°C and about 65°C (see Fig. 10).

The present invention is also related to an isolated and purified nucleotidic sequence from a micro-organism origin, encoding said xylanase polypeptide. Preferably, said micro-organism is selected from the group consisting of bacteria or fungi (including yeast), preferably the *Scytalidium* species fungi, more specifically *Scytalidium acidophilum.*

According to a preferred embodiment of the present invention, said micro-organism is specifically *Scytalidium acidophilum.*

According to the invention, said nucleotide sequence presents more than 60%, preferably more than 70%, 80%, 85%, 90%, 95% more preferably more than 90%, 95% or 98% - 99% homology (or sequence identity) with the sequence SEQ ID NO 11.

According to a preferred embodiment of the present invention, said isolated and purified nucleotide sequence corresponds to the nucleotide sequence SEQ ID NO 11, a variant or a portion thereof encoding a peptide having a xylanase activity.

It is meant by "a portion of the nucleotide sequence SEQ ID NO 11", a fragment of said sequence SEQ ID NO 11 having more than 90 nucleotides, preferably more than 150 nucleotides or more than 300 nucleotides, of said nucleotide sequence and encoding a polypeptide having a xylanase enzymatic activity similar to the xylanase activity of the complete amino-acid sequence SEQ ID NO 8.

Another aspect of the present invention is related to a recombinant nucleotide sequence comprising, operably linked to the nucleotide sequence according to the invention and above-described, one or more adjacent regulatory sequence(s), preferably originating from homologous micro-organisms.

However, said adjacent regulatory sequences may also be originating from heterologous microorganisms.

These adjacent regulatory sequences are specific sequences such as promoters, secretion signal sequences and terminators.

Another aspect of the present invention is related to the vector comprising the nucleotide sequence(s) according to the invention, possibly operably linked to one or more adjacent regulatory sequence(s) originating from homologous or from heterologous micro-organisms.

It is meant by "a vector", any biochemical construct which may be used for the introduction of a nucleotide sequence (by transduction, transfection, transformation, infection, conjugation, etc.) into a cell. Advantageously, the vector according to the invention is selected from the group consisting of plasmids, viruses, phagemids, chromosomes, transposons, liposomes, cationic vesicles or a mixture thereof. Said vector may comprise already one or more of the above-described adjacent regulatory sequence(s) (able to allow its expression and its transcription into a corresponding peptide by said micro-organism).

The present invention is also related to the host cell, preferably a recombinant host cell, "transformed" by the nucleotide sequence or the vector according to the invention above-described.

It is meant by "a host cell "transformed" by the nucleotide sequence or the vector according to the invention", a cell having incorporated said nucleotide sequence or said vector and which does not comprise naturally (originally) said nucleotide sequence. The transformed host cell may be also a cell having incorporated said vector or said nucleotide sequence by genetic transformation, preferably by homologous recombination or other method (recombinant micro-organism).

A "host cell" may be also the original cell comprising the nucleotide sequence encoding the enzyme according to the invention and genetically modified (recombinant host cell) to overexpress or express more efficiently said enzyme (better pH profile, higher extra cellular expression,...).

Preferably, said host cell is also capable of overexpressing (higher expression than the expression observed in the initial micro-organism) said nucleotide sequence or said vector and allows advantageously a high production of an amino acid sequence encoded by said nucleotide sequence or by said vector. The isolated and purified nucleotide sequence according to the invention may be either integrated into the genome of the selected host cell or present on an episomal vector in said host cell.

Advantageously, the recombinant host cell according to the invention is selected from the group consisting of the microbial world, preferably bacteria or fungi, including yeast.

Preferably, said recombinant host cell is Pichia Pastoris, having the deposit number MUCL-43962 (deposit date 12/07/2002).

Preferably, said recombinant host cell is modified to obtain an expression of the xylanase enzyme at high level obtained by the use of adjacent regulatory sequences being capable of directing the overexpression of the nucleotide sequence according to the invention in the recombinant host cell or by increasing the number of nucleotide copies of the sequences according to the invention.

The following description describes also the conditions (culture media, temperature and pH conditions, etc.) for the culture of the host selected for the expression of the xylanase according to the invention. For this purpose, the original production species and/or a suitable host cell transformed with a DNA construct designed to express the said enzyme are present in a suitable growth medium.

According to the present invention, said protein with xylanolytic activity may be isolated from the medium and/or purified. The culture, isolation and purification conditions are derived from conventional methods well-known to persons skilled in the art.

The xylanase enzyme according to the invention may be used in different kinds of industries.

The enzyme with xylanolytic activity of the present invention, purified or not purified, is particularly suited as a bread-improving agent. Bread-improving agents are products which could improve or increase texture, flavour, anti-staling effect, softness, crumb softness upon storage, freshness and machinability, volume of a dough and/or of a final baked product. Preferably, said enzyme with xylanolytic activity increases the specific volume of the final baked product.

"Baked product" intends to include any product prepared from dough, in particular a bread product. Dough is obtained from any type of flour or meal (e.g. based on rye, barley, oat or maize, preferably prepared with wheat or with mixes including wheat.

A further aspect of the present invention relates to the additive effect of said enzyme having xylanolytic activity with other enzymes, in particular with an alpha-amylase, preferably an alpha-amylase from *Aspergillus oryzae.* Said enzyme with xylanolytic activity may be used in combination with other bread-improving agents like enzymes, emulsifiers, oxidants, milk powder, fats, sugars, amino acids, salts, proteins (gluten, cellulose binding site) well known to the person skilled in the art.

According to the present invention, the enzyme with xylanolytic activity, purified or not, shows hydrolytic activities in presence of plant cell wall components. Particularly, said enzyme degrades the wheat cell wall components. Particularly, the degradation activities lead to a decrease of the flour viscosity in the presence of water. Said enzyme may thus advantageously be used in the separation of components of plant cell materials such as cereal components. Particularly, said enzyme may be used to improve the separation of the wheat into gluten and starch by the so-called batter process.

According to the present invention, said enzyme may be used to improve the filtrability and/or decrease the viscosity of glucose syrups obtained from impure cereal starch by subjecting the impure starch first to the action of an alpha-amylase, then to the action of said xylanase. It may also be used in beer brewing when cereal has to be degraded to improve the filtrability of the wort or to re-use the residuals from beer production for e.g. animal feed. Said enzyme may be used in feed to improve the growth rate or the feed conversion ratio of animals such as poultry.

Another application resides in the oil extraction where oil has to be extracted from the plant material such as the corn oil from corn embryos. The enzyme with xylanolytic activity of the present invention may be used in fruit and vegetable juice processing to improve the yield. According to the present invention, said enzyme may be used in all processes involving plant materials or waste materials, e.g. from paper production, or agricultural wastes such as wheat-straw, corn cobs, whole corn plants, nut shells, grass, vegetable hulls, bean hulls, spent grains, sugar beet, and the like.

The effect of the enzyme with xylanolytic activity of the present invention may be further improved by adding other enzymes in combination with said enzyme. Such enzymes may belong but are not restricted to hydrolytic enzymes families such as glucanases, proteases, cellulases, hemicellulases, pectinases. Other enzymes are transglutaminases, oxido-reductases, isomerases, etc.

The enzyme with xylanolytic activity according to the invention may be used under several forms. Cells expressing the enzyme, such as yeast, fungi, archea bacteria or bacteria, may be used directly in the process. Said enzyme may be used as a cell extract, a cell-free extract (i.e. portions of the host cell that has been submitted to one or more disruption, centrifugation and/or extraction steps) or as a purified protein. Any of the above-described forms may be used in combination with one or more other enzyme(s) under any of the above-described forms. These whole cells, cell extracts, cell-free extracts or purified enzymes may be immobilised by any conventional means on a solid support to allow protection of the enzyme, continuous hydrolysis of substrate and/or recycling of the enzymatic preparation. Said cells, cell extracts, cell-free extracts or enzymes may be mixed with different ingredients (e.g. in the form of a dry powder or a granulate, in particular a non-dusting granulate, in a form of a liquid, for example with stabilisers such as polyols, sugars, organic acids, sugar alcohols according to well-established methods). A deposit of biological material MUCL-43962 was done at the Culture Collection of the Université Catholique de Louvain, Place Croix du Sud No.3, on 12 July 2002 according to the requirements of the Budapest Treaty.

The invention will be described in further details in the following examples by reference to the enclosed drawings, without limiting its scope.

### Brief description of the drawings

Figure 1 shows a Southern blot analysis of the *Scytalidium acidophilum* genomic DNA.
Lane 1: molecular weight standard: phage λ restricted by *HindIII.*
Lane 2: 1,5 µg genomic DNA restricted by *EcoRI*
Lane 3: 1,5 µg genomic DNA restricted by *HindIII*
Lane 4: 1,5 µg genomic DNA restricted by *SspI*
Lane 5: 1,5 µg genomic DNA restricted by *XbaI*

Figure 2 represents the complete genetic sequence of the xylanase (SEQ ID NO 11) according to the invention. The start codon (ATG) and the stop codon (TGA) are in bold. The intron of 55bp are in italic.

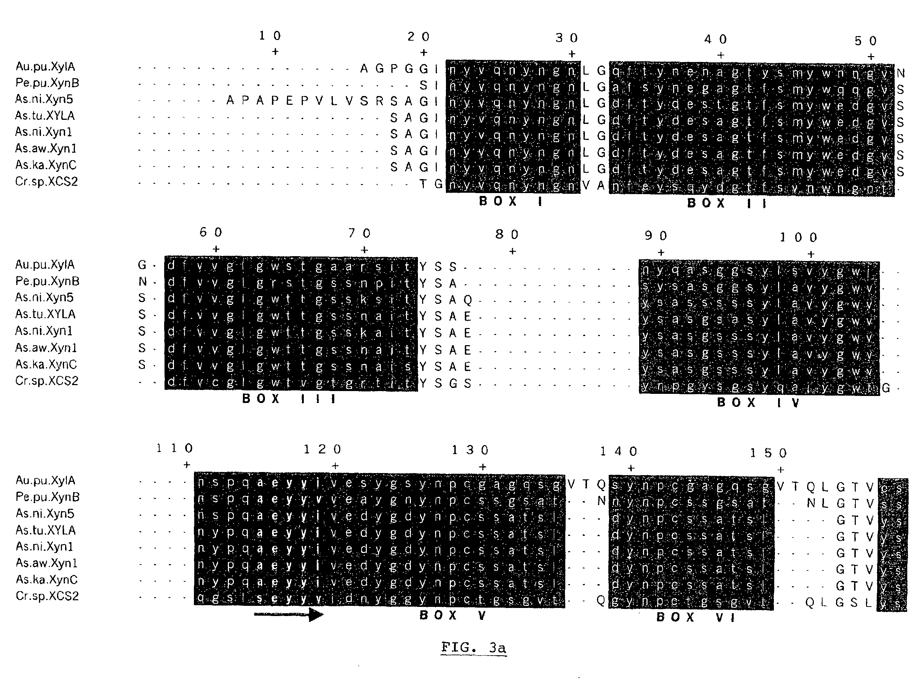

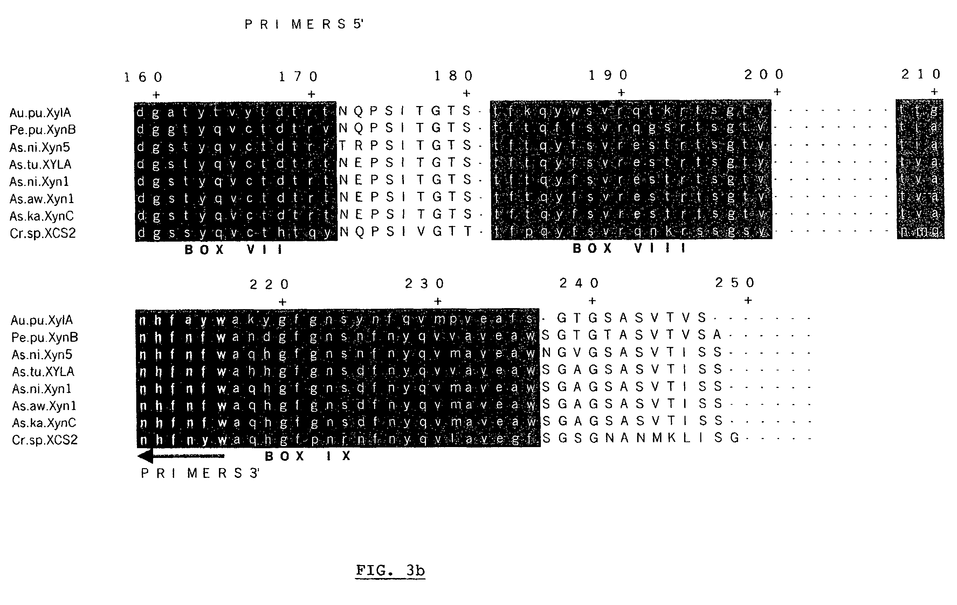

Figure 3a and 3b show the multiple sequences alignments of fungal xylanases. Alignement of 8 amino-acid sequences of xylanases from family G of fungal origin is presented: *Aureobasidium pullulans* (Au.pu.XylA; AAD51950), *Penicillium purpurogenum* (Pe.pu.XYNB; AAK50762), *Aspergillus niger* (As.ni Xyn5; AAA99065), *Aspergillus tubigensis* (As.tu.XYLA;P55331) , *Aspergillus niger* (As.ni.XYN1; P55329), *Aspergillus niger var.awamorii* (As.aw.Xyn1; P55328), *Aspergillus kawachii* (As.ka.XynC;1BK1), *Cryptococcus sp.* (Cr.sp.XCS2;JC4909). Conserved regions are in boxes (I to IX). Amino acid sequences from which the degenerated primers are defined are in bold and underlined with arrows.

Figure 4 shows the construction of pSA1 vector which contains or 225 bp xylanase probe.

Figure 5 represents the complete amino acids sequence of the xylanase (SEQ ID NO 8) according to the invention. The predicted signal peptide is in bold.

Figure 6 represents the sequence of the 5' (SEQ ID NO 12) and the 3' exon 5' (SEQ ID NO 13).

Figure 7 shows the construction of pre-pSA4 vector wich contains the *XYLSA1* gene ORF.

Figure 8 shows the construction of surexpression pSA4 vector wich contains the *XYLSA1* gene ORF in frame with the *AOX1* promoter.

Figure 9 shows the evolution of xylanase activity during induction time.

Figure 10 shows the evolution of xylanase activity depending on temperature.

Figure 11 shows the evolution of xylanase activity depending on pH.

### Detailed description of the invention

### Example 1: Purification of an enzyme with xylanolytic activity from Scytalidium acidophilum

### Strain

Among strains tested that appeared on plates after incubation at 30°C, a particular strain was isolated and identified as *Scytalidium acidophilum.*

Said strain was grown for a week in a liquid medium at 30°C. More precisely, liquid medium was an acidic solution comprising 27 ml H₂SO₄ 37.5N, 6.3 ml H₃PO₄, and 5 ml NH₄OH 32%, mixed with a salt solution comprising 2.5g HKOH, 2g NaOH, 0.1g FeCl₃ and 25g MgCO₃. pH was adjusted to 2 with NaOH. 2 ml of trace solution (0.12g H₃BO₃, 0.5g NaMoO₄.2H₂O, 0.8g CuSO₄.5H₂O, 0.15g MnCl₂.4H₂O) and 3 g of yeast extract were added. Volume was adjusted to 900 ml and solution was autoclaved. 100ml of a 20g/L carbon source solution (glucose or xylan) separately autoclaved was added.

Mycelia were separated from 10 ml culture media by filtration (coffee filter). Supernatant was collected. Grams of mycelia were resuspended in 5 ml of McIlvain's buffer pH3. Mycelia were disrupted by pressurisation at 2 K Bar (Constant cell disruption system, Constant System). After lysis, the mixture was centrifuged 10 min at 12 000 r.p.m.. Soluble fraction was collected. Cell remains were resuspended in 2 ml of McIlvaine's pH3 (insolube fraction).

Activity test was done on the 3 fractions, the supernatant, the soluble fraction and the insoluble fraction.

### Determination of the xylanolytic activity

The xylanolytic activity was determined by measuring the reducing sugars formed from the Beechwood xylan (Sigma). The reducing sugars were revealed with the 2,3-dinitrosalicylic acid (Bailey et al., J. Biotechnol. Vol 23, p 257 (1992)). The reaction was carried out at 40°C in McIlvain's buffer (0.2 M sodium phosphate, 0.1M citric acid, pH3). A 60 µl enzyme sample was incubated with 440 µl of 2.5% birchwood xylan in McIlvain's buffer. The reaction was stopped after 10 min by addition of 500 µl DNS solution and boiled for 15 min. The solution was then cooled and centrifuged for 5 min at 12 000 g. The absorption at 575 nm was measured relative to a xylose standard curve (µmol/ml*min) . One international unit (IU) of activity was defined as the amount of enzyme required to release 1 µmol of reducing sugar per minute.

Two cultures are done, one with 2% glucose, the orther with 1% glucose and 1% Beechwood xylan (Sigma, X-4252). For each culture, mycelia were separated from a 10 ml aliquot by centrifugation 10 min at 5000rpm. Supernatant is clarified by filtration (coffee filter and put on ice. Dtt at a final concentration of 0,5 mM and PMSF at a final concentration of 1 mM was added.

Mycelia are washed with 10 ml of McIlvain's buffer pH3. After centrifugation mycelia are resuspended in 6 ml of McIlvain's buffer pH3. Mycelia were disrupted by pressurisation at 2 Bars. After lysis, the mixture was centrifuged 10 min at 10 000 r.p.m.. Soluble fraction was collected. Cell remains were resuspended in 2 ml of McIlvaine's pH3 (insolube fraction).

For each culture, activity test was done on the 3 fractions, the supernatant, the soluble fraction and the insoluble fraction (table 1).

**Table 1**

| | Activité (UI/ml) |
|---|---|
| Supernatant glucose 2% | 0,48 |
| Soluble fraction glucose 2% | 0 |
| Insoluble fraction glucose 2% | 0 |
| Supernatant glucose 1%/xyllan 1% | 8,25 |
| Soluble fraction glucose 1%/xyllan 1% | 0 |
| Insoluble fraction glucose 1%/xyllan 1% | 4,76 |

### Example 2: Cloning of a gene coding for an enzyme with xylanolytic activity

### • Materials and methods

### 1. DNA library

DNA manipulations were carried out by using standard procedures (Maniatis et al., 1989, Molecular cloning: a laboratory manual (second edition). Cold Spring Harbor Laboratory, Cold Spring Harbor, NY).

DNA library was constructed as follows. Genomic DNA from *S. acidophilum* was obtained with "Wizard Genomic DNA Purification Kit" (Promega). It was partially digested with Sau3A. The digested genomic DNA was separated by electrophoresis. Two populations of fragments (of between 3 and 5 kb for the first population and of between 5 and 10 kb for the second population ) were purified out of agarose using a DNA extraction Kit (MBI Fermentas, K0513) on a final volume of 10 µl.

Each population of purified fragments were inserted by ligation in the pUC19 vector (Biolabs). For this purpose, 18µg of pUC19 plasmid DNA was firstly digested with 120 units of *Bam*HI in 50 µl at 37°C for 2 hours. It was then dephosphorylated with 10 unit of Phosphatase alkaline schrimp (Roche, Cat N°: 1758250) in 70 µl at 37°C for 10 min, then at 65°C for 15 min. The sample was subsequently purified from enzymes using a DNA extraction Kit (MBI Fermentas, K0513) in a final volume of 20 µl. The ligation was performed using 3 µg of digested pUC19, 9 µl of purified genomic DNA fragments, 3 units of T4 DNA ligase (Roche, Cat N°: 481220), 3 µl of T4 DNA ligase buffer in final volume of 30 µl.

Of each ligation, 6 times 4 µl were electroporated into 40 µl of Escherichia Coli host DH10b (Gibco BRL). After electroporation, transformed cells were plated on Luria Bertani plates supplemented with 100 µg/ml ampicillin (Roche, N° Cat 835242). From this transformation, 38 000 independent clones were obtained.

### 2. Synthesis of specific probes

Alignment of amino-acid sequences of xylanases from family G (which includes enzymes from several fungi) was done by software Matchbox (DEPIEREUX *et al.*, 1997, "Match-Box_server: a multiple sequence alignment tool placing emphasis on reliability" Comput Appl Biosci. Jun; 13(3) : 249-56.) , as illustrated on figure 3. The conserved regions are shown as shadow boxes.

Oligonucleotides were synthesised by GENSET (Paris, France). Nucleotidic sequences were determined by double strand sequencing using the dideoxy method (Sanger et al., 1977, DNA sequencing with chain-terminating inhibitors, Proc Natl Acad Sci. Dec; 74(12): 5463-7). The experiments were performed on a fluorescent DNA sequencer (ABI PRISM 377 DNA Sequencer) with the DNA sequencing kit, Big Dye Terminator Cycle Sequencing (Perkin Elmer, Part No 43 03 152).

### • Results

### 1. cloning of specific probes

8 amino-acid sequences of fungal xylanases from family G were aligned (figure 3a and 3b), from which four degenerated primers for xylanase gene were designed in box VI:

SEQ ID NO.10: 5'-GCNGARTAYTAYATHGT-3',

SEQ ID NO.20: 5'-GCNGARTAYTAYGTNGT-3'

SEQ ID NO.30: 5'-TCNGARTAYTA YGTNAT-3'

SEQ ID NO.40: 5'-AGYGARTAYTAYGTNAT-3'

Four degenerated primers for xylanase gene were designed in box IX:

SEQ ID NO.50: 5'-CCARTANGCRAARTGRTT-3'

SEQ ID NO.60: 5'-CCARTARTTRAARTGRTT-3'

SEQ ID NO.70: 5'-CCARAARTTRAARTGRTT-3'

SEQ ID NO.80: 5'- CCANGCRTCRAARTGRTT-3'

With these primers PCR was carried out on genomic DNA. With the pair SEQ ID NO.10 and SEQ ID NO.50, a fragment of about 300 base pairs was amplified from genomic DNA. Sequence for this fragment was established. Sequence analysis done with the BLAST software showed strong homology (88%) with several xylanases already sequenced and published.

Specific primers SEQ ID NO.90 and SEQ ID NO.100 have been determined and used to amplify a 225 bp probe from genomic DNA. The sequence of said specific primers is the following:

SEQ ID NO.90: 5'-GCGGAATACTACATCGTTG-3'

SEQ ID NO.100: 5'-CCAGTAGGCGAAATGGTTGC-3'

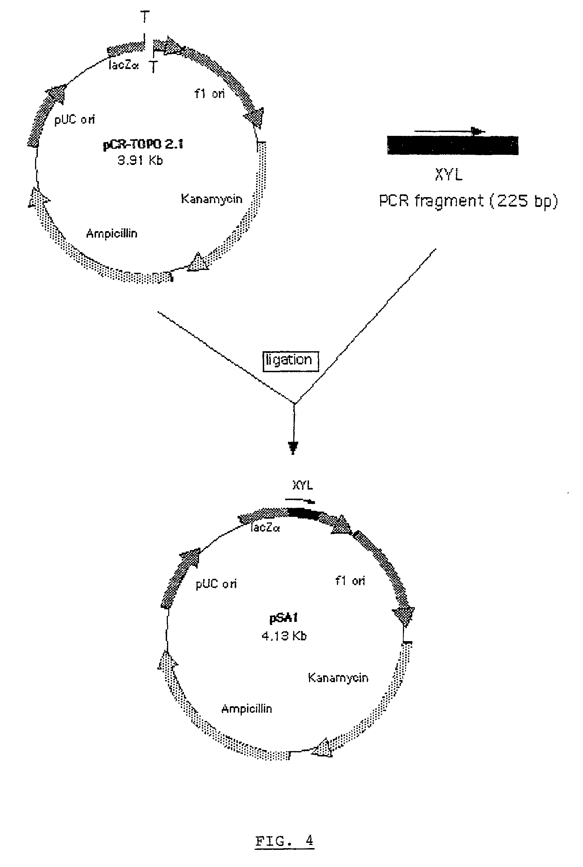

Said 225 bp probe was cloned in the vector pCR2.1 TOPO (Invitrogen, Leek, Netherlands) so as to give the plasmid pSA1 (see figure 4) .

### 2. Southern blot

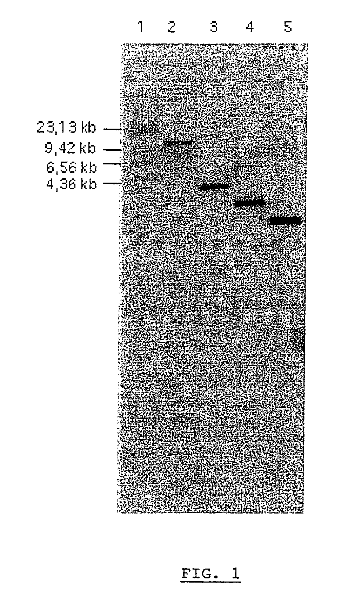

A southern blot was done with the 225 bp probe. Genomic DNA was digested with *Eco*RI, *Hin*dIII, *Ssp*I and *Xba*I. Autroradiogram presented at figure 1 show that the probe has a good affinity.

### 3. Cloning and characterisation of the xylanase gene

60 000 colonies from genomic library were screened by hybridisation on colonies using the specific probe labelled with ³²P. Six positive clones were selected and sequenced. Only one contained a xylanase sequence. This clone was named pSA2 and contained a 3 200 bp insert.

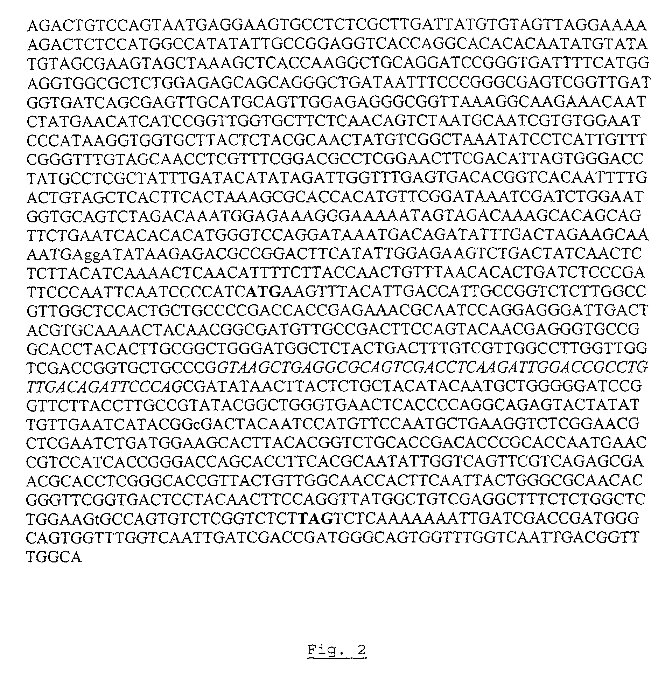

The sequence of 1551 bp, referenced as SEQ ID NO.11 (figure 2), was determined on both strands by primer walking. From the nucleotide sequence, DNA strider program (DNA Strider 1.2, C program for DNA and Protein Sequences Analysis, designed and written by Christian Marck, CEA France) allowed the identification of an open reading frame of 673 bp with a 55 bp intron which encoded a 205 amino-acid polypeptide referenced as SEQ ID NO.8 (see figure 5). Search for homology sequences by screening the NR Database with the BLASTP (Altschul SF, Gish W, Miller W, Myers EW, Lipman DJ, (1990) Basic local alignment search tool, J Mol Biol **215**(3): 403-410) revealed that the deduced protein sequence was clearly a Family 11 xylanase sequence (69% identity and 80% similarity with *Xyn* 1 of *Aspergillus Niger*).

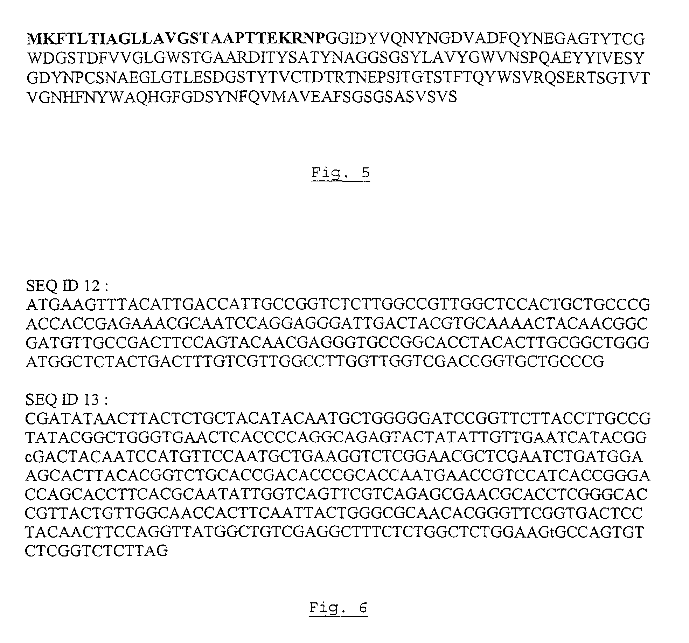

The localisation of the intron was confirmed by the presence of the putative Lariat-formation internal sequence and with the definition of 5' and 3' consensus splice-junction sequences ("GT-AG" rule). The sequences SEQ ID NO.12 and SEQ ID NO.13, represented on figure 6, correspond to the sequences encoding the 5' exon and the 3' exon, respectively.

The PROT PARAM TOOL EXPASY program (http://www.expasy.ch/tools/protparam.html) indicated a theoretical acidic isoelectric point (pI) value of 4.21 and a theoretical molecular weight (PM) of 21.8 kD. A putative signal peptide was localised on the 26 first amino acids (see figure 5). The deduced mature protein has a theoretical acidic isoelectric point (pI) value of 3.94 and a theoretical molecular weight (PM) of 19.1 kD

### Example 3: Expression of xylanolitic gene XYLSA1 in Pichia pastoris

### Bacterial and fungal strains

The strain *Echerischia coli* DH10B (Gibco BRL) was used as a host for contruction and routine propagation xylanase expression vectors in this study. Wild type strain X-33 of *Pichia pastoris* from Invitrogen (Leek, Netherlands) was used for expression.

### Plasmids

Plasmid pSA2 was the plasmid containing the *XYLSA1* gene isolated from the library (see above). Plasmid pSKoriT restricted by EcoRV was used as an intermediary plasmid. Plasmid pPICZαA (Invitrogen, Leek, Netherlands) was the plasmid selected for expression. Said expression plasmid contained a yeast invertase signal sequence as well as the AOX1 promoter and terminator sequences.

### Obtention of the coding sequence

The two 5'exon and 3'exon, referenced hereabove as SEQ ID NO.12 and SEQ ID NO.13 respectively, were amplified by PCR.

For the 5'exon (SEQ ID NO.12), two synthetic oligonucleotides were chosen: SEQ ID NO.15 and SEQ ID NO.16, with the following sequences:

SEQ ID NO.15: 5'- ATATATATCTCGAGAAAAGAATGAAGTTTACATTGACC-3'

SEQ ID NO.16: 5'-CGGGCAGCACCGGTCGACCA-3'

SEQ ID NO.15 corresponds to the 5' oligonucleotide and contains a XhoI site, a Kex2 signal cleavage sequence and the ATG initiation codon of the xylanase gene. SEQ ID NO.16 corresponds to the 3' oligonucleotide.

For the 3'exon, two synthetic oligonucleotides were also chosen: SEQ ID NO.17 and SEQ ID NO.18, with the following sequences:

SEQ ID NO.17: 5'-CGATATAACTTACTCTGCTAC-3'

SEQ ID NO.18: 5'-ATATATATTCTAGACTAAGAGACCGAGACACT-3'.

SEQ ID NO.17 corresponds to the 5' oligonucleotide, while SEQ ID NO.18 corresponds to the 3' oligonucleotide and contains a *Xba*I site.

Conditions for performing the PCR were the following: 3 min at 94°C; 30 times (1 min at 94°C; 0.5 min at 55°C, 10 min at 72°C) in presence of the Pfu DNA polymerase (Stratagene, Cat N°: 600250). The resulting PCR fragments were purified using a DNA extraction Kit (MBI Fermentas, K0513) in a final volume of 17 l.

The two amplified exons were ligated as follows. 16 µl of the two fragments were phosphorylated with one unit of Polynucleotide Kinase (30 min at 37°C). Each phosphorylated fragment was precipitated with ethanol and resuspended in 10 µl of bi-distillated water. The two fragments were ligated to each other with 1.5 unit of T4 DNA Ligase (4 hours at room temperature). This mixture was then precipitated with ethanol and resuspended in 15 µl of bi-distillated water.

The coding sequence was amplified from the ligation mixture with oligonucleotides SEQ ID NO.15 and SEQ ID NO.18 in the same conditions as described above.

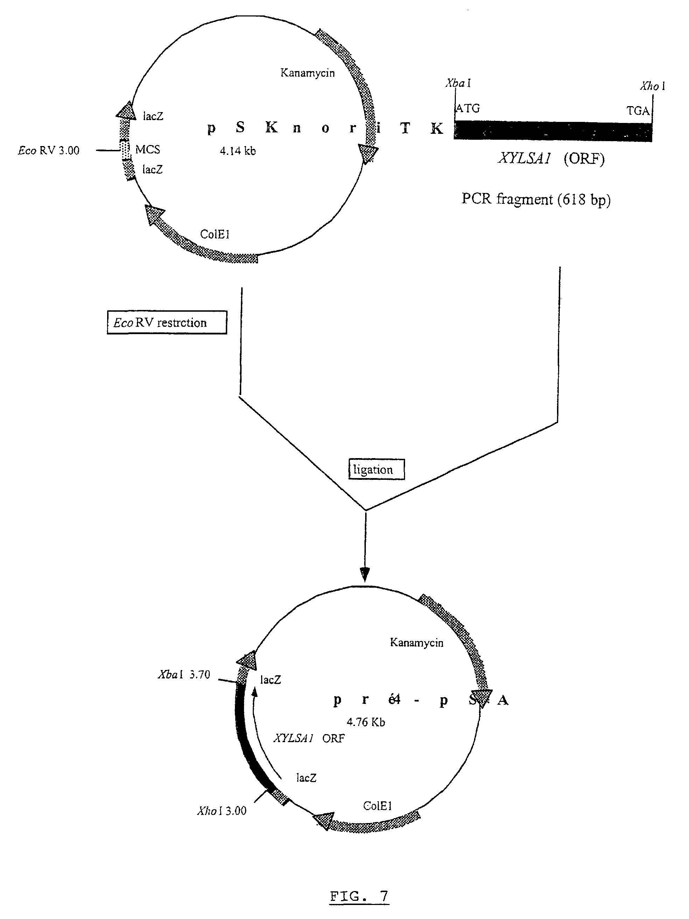

This PCR fragment was inserted blunt end in the plasmid pSKoriT (Stratagene) restricted with *Eco*RV. The resulting plasmid was named pre-pSA4 (figure 7).

Said resulting plasmid pre-pSA4 was restricted with the restriction enzymes *Xba*I and *Xho*I. This fragment was then inserted in the *Xho*I and *Xba*I restriction sites of the expression vector pPICZαA (Invitrogen, Leek, Netherlands). 4 µl of the ligation mixture was transformed into 40 µl of *E.Coli* host DH10B (Gibco BRL) by heat shock. After transformation, cells were plated on low salt Luria Bertani plates (For 1 liter: 10 g Tryptone, 5g NaCl, 5g Yeast Extract, 15g Agar) supplemented with zeocin (Invitrogen, Leek, Netherlands) at 25 µg/ml.

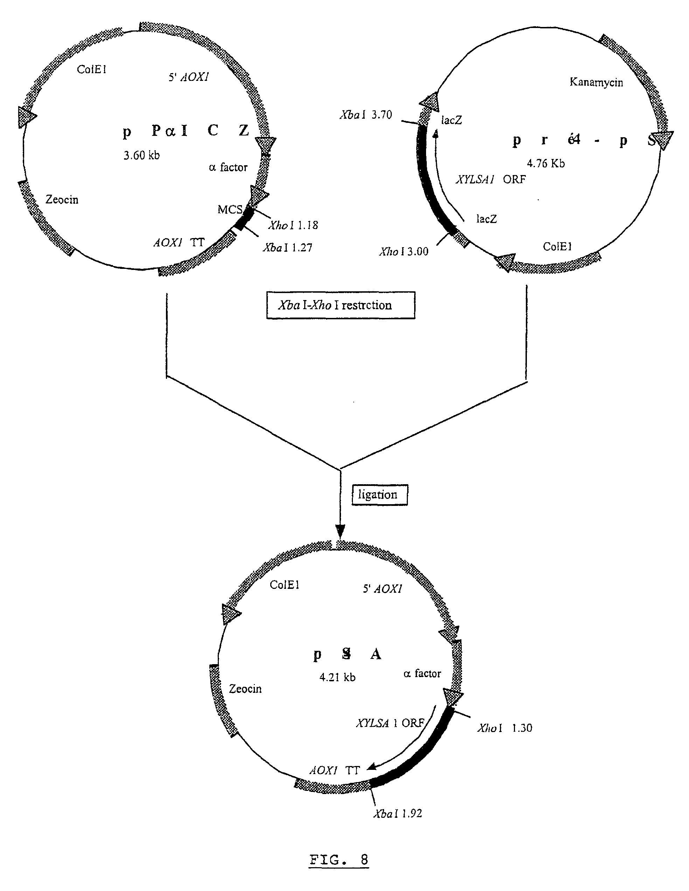

The resulting expression plasmid was named pSA4. In this plasmid, the *XYLSA1* coding sequence was in fusion with the yeast invertase signal sequence and under the control of the *AOX1* promoter (see figure 8). The PCR amplicon was checked by sequencing.

### Transformation of Pichia pastoris strain

The wild type strain X-33 of *Pichia pastoris* was transformed by electroporation with the pSA4 plasmid linearised with the ScaI endonuclease according to the protocol described by Invitrogen (EasySelect™ *Pichia* Expression Kit, A manual of methods for expression of recombinants proteins using pPICZ and pPICZa in *Pichia pastoris,* Catalog no K1740-01). Transformants were selected on YPD plates with zeocin (100 µg/ml).

### Analysis of Pichia pastoris transformants

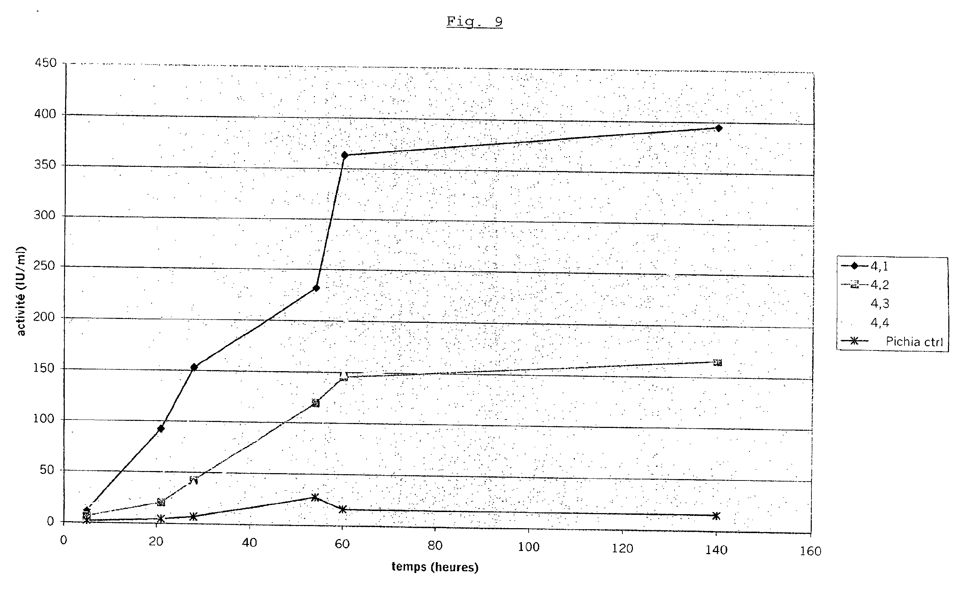

Integration was tested by PCR on 10 transformants, all were positive. Four transformants (named pSA4.1, pSA4.2, p.SA4.3, pSA4.4) were screened for xylanase activity in small-culture analysis. They were grown under non-induced conditions in 25 ml of BMGY medium (1% yeast extract, 2% peptone, 100 mM potassium phosphate, pH6, 1.34% YNB, 4 10⁻⁵% biotin, 1% glycerol) for 16 hours at 30°C. Cells were harvested by centrifuging at 3000 g for 5 minutes. Cell pellet was resuspended to an optical density at 600 nm equal to 1 in methanol medium BMMY (1% yeast extract, 2% peptone, 100 mM potassium phosphate, pH6 1.34% YNB, 4 10⁻⁵% biotin, 0,5% methanol; induced conditions). Culture was returned to incubator to continue growth. 100% methanol was added to a final concentration of 0.5% very 24 hours to maintain induction. Aliquots of the culture supernatant (500 µl) obtained at different times (5,21,28,45,54,60 hours) of induction were subjected xylanase activity assay and to SDS-PAGE. As illustrated on figure 9, measured xylanase activity was growing up as a function of incubation time in the four clones, but in a different manner.

### Scaling-up of the production of xylanase

The best clone (PSA4.1) on basis of activity was used to inoculate a 5 litres fermentator (BIOFLOW 3000 New Brunswick scientific). Fermentation proceeded in two phases. The first phase was consisting in providing an important biomass (glycerol fed-batch phase), and the second phase was the induction phase whereby xylanase was produced (methanol fed-batch phase).

In order to prepare inoculum, growing culture was carried out by inoculating 500 ml of BMGY in shaker flask with a colony of clone pSA4.1, flask being placed on a shaker for 24 hours at 30°C and 200 r.p.m.

The cultivation was carried out at 30°C, 500 r.p.m (normal bladder stirrer). The fermentation broth was aerated at 0.5 (v/v/min) and the pH was kept automatically at 3 (NaOH 2N). Cells were separated from the supernatant by filtration on membrane filtration unit (Corning, Lab. Scale membrane, V-2, 0,2µ, product code: N1L2(0,2) (V2); saniterying housing: H1L2-F-316). From 2 litres supernatant, xylanase was dialysed and concentrated to 300 ml using ultrafiltration. Protein concentration, 2,6g/l, was estimated by the bicinchronic acid method (Pierce, Rockford, IL, USA). Xylanase activity was 1809 UI/ml. Specific activity was 1807/2,6 UI/mg, 695 UI/mg.

### Example 4: Characterisation of the enzyme with xylanolytic activity from S. acidophilum

### Purification of the recombinant enzyme with xylanolytic activity expressed in P. pastoris

The enzyme with xylanolytic activity expressed in *P. pastoris* was secreted and analysed on SDS-PAGE gel. The protein purified in order to separate it from the traces of α-amylase present in the culture supernatants of the transformants. The proceedings was the same as mentioned in Example 1 using affinity column purification methods. The active fractions with xylanolytic activity were pooled and kept for further analysis.)

### Optimum pH and temperature

The pH and temperature dependence of the activity for the xylanolytic enzyme secreted by *one P. pastoris* transformant was analysed. The activity was measured in a McIlvain's buffer (0.2 M sodium phosphate, 0.1M citric acid, pH 3,4) at various temperature (Fig. 10).

The maximum activity was observed around 50°C. At this temperature, the optimum pH was about 4 (figure 11).

### Example 5: Delignification of kraft pulp

The Kappa number is the volume of 0.1N potassium permanganate solution consumed by 1 gram of moisture-free under specific conditions as disclosed in TAPPI test methods (APPI, Atlanta GA, vol.1, 1998 "Kappa number of pulp-T236cm85).

Hardwood kraft pulp at 9% was incubated 2 hours at 60°C in 50 mM acetate buffer pH3 with 100 UI of xylanase /gr of dry pulp. During incubation, liberation of lignin is followed by absorbance at 280nm (table 2). Two experiments were done.

**Table 2**

| | DO 280 | DO 280 | DO 280 | DO 280 |
|---|---|---|---|---|
| | (0 min) | (45 min) | (100 min) | (120 min) |
| Experiment 1 | | | | |
| Pulp without xylanase | 0,391 | 0,365 | 0,383 | 0,376 |
| Pulp + xylanase | 0,56 | 1,463 | 1,706 | 1,744 |

| Experiment 2 | | | | |
|---|---|---|---|---|
| Pulp without xylanase | 0,282 | 0,289 | 0,331 | 0,343 |
| Pulp + xylanase | 0,715 | 1,22 | 1,1362 | 1,518 |

It can be seen from Table 2 that liberation of lignin is less important in the presence than in the absence of xylanase. The xylanase according to the invention allows a delignification of kraft pulp.

After 2 hours of incubation, pulp was washed 3 times with 50 mM acetate buffer pH3 and the kappa number was determined. The results obtained for 3 independent experiments are presented in table 3.

**Table 3**

| | Kappa indic | Kappa indic | Kappa indic |
|---|---|---|---|
| | Experiment 1 | Experiment 2 | Experiment 3 |
| Pulp without xylanase | 10,5 | 11,2 | 10,4 |
| Pulp + xylanase | 8 | 7,1 | 7,6 |
| Delta Kappa | 2,5 | 4,1 | 2,8 |

These obtained results show that consumption of potassium permanganate solution is less important when the xylanase is present, thereby confirming the results obtained in Table 2 concerning the ability of the xylanase to delignify hardwood kraft pulp.

## Claims

1. An isolated and purified polypeptide with xylanolytic activity having more than 70% of sequence identity with the amino acid sequence SEQ ID NO 8.

2. The polypeptide according to claim 1, having more than 80%, preferably more than 90% of sequence identity with the amino acid sequence SEQ ID NO 8.

3. An isolated and purified polypeptide having an amino acid sequence corresponding to the amino acid sequence of SEQ ID NO 8, a variant or a portion thereof having a xylanolytic activity.

4. The polypeptide according to any of the preceding claims, which presents an optimum enzymatic activity at a pH comprised between about 3.5 and about 4.5 and at a temperature comprised between about 45 and about 60°C.

5. An isolated and purified nucleotide sequence encoding the polypeptide according to any one of the preceding claims.

6. An isolated and purified nucleotide sequence which encodes a polypeptide having a xylanolytic enzymatic activity, and which presents more than 70% of sequence identity with the nucleotide sequence SEQ ID NO 11.

7. The isolated and purified nucleotide sequence according to claim 6, which presents more than 80%, preferably more than 90%, of sequence identity with the nucleotide sequence SEQ ID NO 11.

8. An isolated and purified nucleotide sequence corresponding to the nucleotide sequence SEQ ID NO 11, a variant or a portion thereof encoding a polypeptide having a xylanolytic activity.

9. A recombinant nucleotide sequence comprising, operably linked to the nucleotide sequence according to any one of the claims 5 to 8, one or more adjacent regulatory sequence(s), preferably originating from homologous microorganisms.

10. A vector comprising the nucleotide sequence according to any one of the claims 5 to 9.

11. The vector according to claim 10, being a plasmid incorporated in *Escherichia coli* and having the deposit number MUCL-43962.

12. A recombinant host cell transformed by the nucleotide sequence according to any one of the claims 5 to 9 or the vector according to claim 10 or 11.

13. The recombinant host cell according to claim 12, which is selected from the group consisting of bacteria or fungi, including yeast, preferably the strain Pichia Pastoris MUCL-43962.

14. A solid support fixing an element selected from the group consisting of the cell according to claim 12 or 13, a cell extract of the cell according to claim 12 or 13 and/or the isolated and purified polypeptide with xylanolytic activity according to any one of the claims 1 to 4.

15. Use of the recombinant host cell according to claim 12 or 13 or of the polypeptide with xylanolytic activity according to any one of the claims 1 to 4, for the degradation of plant cell wall components.

16. Use according to claim 15 in the preparation of fruits, legume juices, beer, paper, starch, gluten or vegetable oil.

17. Use according to claim 15 in the treatment of wastes, preferably agricultural wastes or wastes from paper mills.

18. Use according to claim 15 in baking processes, especially for increasing the volume of baked products.

19. Use according to claim 15 in starch-gluten separation processes.
